# EUROPÄISCHE PATENTSCHRIFT

(11) **EP 3 215 619 B1**
(45) Veröffentlichungstag und Bekanntmachung des Hinweises auf die Patenterteilung: **04.01.2023**
(21) Anmeldenummer: 15837175.7
(22) Anmeldetag: 09.11.2015
(51) Int. Cl.: C07H 1/08, C12N 15/10

(54) **VERFAHREN ZUR SELEKTIVEN GRÖSSENFRAKTIONIERTEN TRENNUNG UND ISOLIERUNG VON NUKLEINSÄUREMISCHUNGEN**
METHOD FOR SIZE-SELECTIVE SEPARATION AND ISOLATION OF NUCLEIC ACID MIXTURES
PROCÉDÉ DE SÉPARATION ET D'ISOLEMENT SELON LA CATÉGORIE DIMENSIONNELLE DE MÉLANGES D'ACIDES NUCLÉIQUES

(30) Priorität: 07.11.2014 DE 102014222810
(43) Veröffentlichungstag der Anmeldung: 13.09.2017
(73) Patentinhaber: IST Innuscreen GmbH, 13125 Berlin (DE)
(72) Erfinder: HILLEBRAND, Timo, 15366 Hoppegarten (DE)
(74) Vertreter: Wehlan, Martin
(86) Internationale Anmeldenummer: PCT/EP2015/076075
(87) Internationale Veröffentlichungsnummer: WO 2016/071535

(56) Entgegenhaltungen:
- EP-A1- 2 128 169
- WO-A1-2013/045434
- WO-A1-2013/181651
- WO-A1-2014/122288

## Beschreibung

Die Erfindung betrifft ein neuartiges Verfahren zur größenfraktionierten Trennung und Isolierung von Nukleinsäuren.

Die Erfindung zielt insbesondere auf solche Anwendungen, bei denen es gewünscht ist, dass nur Nukleinsäuren eines ausgewählten Größenspektrums isoliert werden sollen, damit spezifische downstream-Applikationen effizienter durchgeführt werden können. Die Erfindung ist aber auch dafür geeignet, wenn Anwender unterschiedliche Nukleinsäurefraktionen aus einer Probe analysieren wollen.

Für die Aufreinigung und Rückgewinnung von Nukleinsäuren, insbesondere von DNA bzw. von DNA-Fragmenten, existiert heute eine Vielzahl von kommerziell verfügbaren Kits.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA-Bande enthaltenden Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und abschließend von den Trägerpartikeln abgelöst.

Das physiko-chemische Prinzip dieser Form der spezifischen Anbindung von Nukleinsäuren an mineralische Trägermaterialien soll dabei in der Störung übergeordneter Strukturen des wässrigen Milieus bestehen, durch welche die Nukleinsäuren an der Oberfläche von mineralischen Materialien, insbesondere von Glas-bzw. Silicapartikeln adsorbieren. Die Störung der übergeordneten Strukturen des wässrigen Milieus erfolgt dabei immer unter Anwesenheit chaotroper Ionen und ist bei hohen Konzentrationen dieser fast quantitativ. Seit 1998 ist zusätzlich bekannt, dass eine Anbindung von Nukleinsäuren an mineralische Träger auch gänzlich ohne chaotrope Salze enthaltende Puffer möglich ist. So wird in der Offenlegungsschrift WO2007/036564 A2 offenbart, dass auch sog. antichaotrope Salze enthaltende Puffer eine spezifische Adsorption von Nukleinsäuren an mineralische Träger erlauben, wobei der Prozess der Isolierung der Nukleinsäuren in Analogie zum bekannten Verfahren auf der Basis einer chaotropen Chemie umgesetzt wird. Auch diese Bindungschemie wurde nachfolgend optimiert und verfeinert (WO2007/060248 A1).

In der Druckschrift WO 01/62976 A1 wird ein Verfahren offenbart, welches die Aufreinigung von Nukleinsäuren aus unterschiedlichen Reaktionsansätzen unter Zugabe von unterschiedlichen Alkoholen, deren nachfolgender Präzipitation an spezielle feste Phasen (Membranen mit spezifischen physikalischen Charakteristika), Waschschritten mit alkoholischen Puffern und der finalen Elution der Nukleinsäuren mittels Wasser beschreibt.

Ebenso beschreiben die Patenschriften US 5405951 A und EP 0512767 B1 die Isolierung von Nukleinsäuren durch Inkubation der Nukleinsäure enthaltenden Probe mit einem Alkohol und der nachfolgenden Inkubation der Probe mit einem mineralischen Material. Die Elution der Nukleinsäuren erfolgt über die Zugabe von auf 60°C erwärmtem Wasser.

In der Patentschrift DE 10253351 B4 wird offenbart, dass die Aufreinigung und Rückgewinnung von Nukleinsäuren dadurch erfolgt, dass man die Nukleinsäure enthaltende Lösung mit Zusätzen so einstellt, dass sie monovalente und multivalente Kationen sowie einen Alkohol enthält, sie danach mit der festen Phase in Kontakt bringt, den Träger anschließend ggf. wäscht und die Nukleinsäure von der festen Phase löst. Als monovalente Salzkomponenten werden Ammoniumchlorid, Natriumchlorid und/ oder Kaliumchlorid verwendet und als multivalente Salzkomponente Magnesiumchlorid, Calciumchlorid, Zinkchlorid und/ oder Manganchlorid.

Es wird offenbart, dass gerade die Kombination eines monovalenten und eines multivalenten Salzes dazu führt, dass Nukleinsäuren an feste Phasen adsorbieren, wobei die dazu notwendigen Ionenstärken nur sehr gering sein müssen. Dies hat den Vorteil, dass ggf. bisher immer notwendige Waschschritte nicht mehr benötigt werden und sich somit die Verfahren zur Isolierung von Nukleinsäuren deutlich verkürzen und vereinfachen lassen.

In der Offenlegungsschrift WO2007/065934 A1 wird ein Verfahren offenbart, welches ebenfalls eine Aufreinigung von lang- und kurzkettigen DNA-Fragmenten erlaubt, wobei durch die Verwendung von Salzen der Zitronensäure Puffer eingesetzt werden, die nur geringe Ionenstärken enthalten, so dass ebenfalls Waschschritte nicht mehr notwendig sind.

Alle diese verschiedenen Verfahren zeigen eine Gemeinsamkeit. Sie erlauben immer die Isolierung einer in einer Probe enthaltenen Gesamtnukleinsäure. Dies bedeutet, befindet sich in einer Probe ein Gemisch aus lang- und kurzkettigen Nukleinsäuren, so wird immer dieses Gemisch an Nukleinsäuren aufgereinigt. Es erfolgt keine Trennung oder differenzielle Extraktion von lang- und kurzkettigen Nukleinsäure-Fragmenten bzw. eine gezielte Aufreinigung von Nukleinsäuren, welche sich in einem gewünschten Größenspektrum befinden.

Moderne diagnostische Fragestellungen definieren heute die Notwendigkeit, aus einer Nukleinsäuren enthaltenen Probe selektiv bestimmte Nukleinsäurefraktionen an- bzw. abzureichern bzw. diese Fraktionen differenziert vorliegen zu haben. Als Nukleinsäurefraktion im Sinne der vorliegenden Erfindung ist eine Größenfraktion der Nukleinsäure gemeint, d.h. kurzkettige, längerkettige oder langkettige Nukleinsäuren.

Die Anreicherung von kurzkettigen Nukleinsäuren und damit verbunden eine Abreicherung von längerkettigen und langkettigen Nukleinsäuren aus einer Probe spielt in der modernen Prenataldiagnostik eine wesentliche Rolle. Hierbei geht es um eine gewünschte größenfraktionierte Trennung frei zirkulierender maternaler DNA von frei zirkulierender fötaler DNA im Blut von Schwangeren. Die Nutzung frei zirkulierender fötaler DNA für eine Prenataldiagnostik (z.B. Nachweis von Trisomien) hat den Vorteil, dass eine invasive Untersuchung (Amniozentese oder Chorionzottenbiopsie) nicht notwendig wäre, welche für den wachsenden Föten immer ein Risiko bedeutet. Dem Fachmann ist aber auch bekannt, dass der Anteil an frei zirkulierender DNA fötalen Ursprungs im Blut der Mutter generell sehr viel geringer ist, als der Anteil der frei zirkulierenden DNA der Mutter. Durch den großen Überschuss maternaler DNA wird die Untersuchung von Targetsequenzen des Föten deutlich erschwert, dies insbesondere bei der DNA-Sequenzierung.

Darüber hinaus ist der Anteil der frei zirkulierenden DNA generell immer sehr gering, was zusätzlich eine Diagnostik erschwert, da nicht immer eine ausreichende Menge an DNA bereitgestellt werden kann. Hier wäre es wünschenswert, mehr Probenvolumen für eine Extraktion einsetzen zu können. Neben der Prenataldiagnostik spielt die Isolierung von frei zirkulierender Nukleinsäure aus Körperflüssigkeiten eine immer größere Rolle. Dies betrifft die molekulare Tumordiagnostik, die Untersuchung von Transplantatabstoßungsreaktionen u.v.m. Auch bei diesen Forschungsfeldern ist es immer mehr von Interesse, eine selektive größenfraktionierte Extraktion von Nukleinsäuren aus einer Probe durchführen zu können. Wichtig ist wiederum auch, dass man ausreichende Mengen an Nukleinsäuren isoliert und aus diesem Grunde größere Probenvolumina einfach und schnell bearbeiten kann. Wichtig ist auch, generell zu untersuchen, wie sich die Größenverteilung von Nukleinsäuren in einer Probe darstellt, da daraus Rückschlüsse über den Ursprung von frei zirkulierender DNA gezogen werden können.

In der WO2009/146776 A2 ist ein Verfahren offenbart, welches die Isolierung/ Aufreinigung von kurzkettigen Nukleinsäuren aus einem nukleinsäurehaltigen Ausgangsmaterial beschreibt.

Dabei kommen zwei Varianten zum Einsatz. Die Aufreinigung von insbesondere kurzkettigen Nukleinsäuren erfolgt dadurch, dass das Ausgangsmaterial mit einer chaotropen Verbindung, mit Isopropanol sowie einem nukleinsäurebindenden Trägermaterial in Kontakt gebracht wird. Dabei soll der Alkohol in einer Konzentration von ≥ 5% (v/v) und kleiner gleich ≤ 40% (v/v) vorliegen. Dadurch sollen kurzkettigere Nukleinsäuren effizienter an das Trägermaterial binden. Die gebundenen Nukleinsäuren können am Trägermaterial verbleiben, können aber auch eluiert werden. Nach Daten der Druckschrift sollen kurzkettige Nukleinsäuren unter diesen Bindungsbedingungen besonders effizient aufgereinigt werden (Kombination von chaotroper Verbindung mit Isopropanol) und sogar eine Anreicherung von kurzkettigen Nukleinsäuren gegenüber längerkettigen Nukleinsäuren möglich sein. Das Verfahren beschreibt aber keine wirkliche Trennung von kurzkettigen und längerkettigen Nukleinsäuren. Alle Fraktionen werden aufgereinigt. Um eine Trennung von kurzkettigen und langkettigen Nukleinsäuren durchzuführen, wird in dieser Schrift ein zweites Verfahren beschrieben. Bei diesem Verfahren wird das Ausgangsmaterial mit einer chaotropen Verbindung, mit einem verzweigten und/ oder unverzweigten Alkohol sowie einem nukleinsäurebindenden Trägermaterial in Kontakt gebracht, wobei der Alkohol in einer Konzentration von ≤ 30% (v/v) vorliegt. Danach wird der Durchbruch oder Überstand aus dem ersten Schritt mit einer chaotropen Verbindung, mit einem verzweigten und/ oder unverzweigten Alkohol sowie einem nukleinsäurebindenden Trägermaterial in Kontakt gebracht, wobei der Alkohol in einer Konzentration von ≥ 5% (v/v) vorliegt. Es wird ausgeführt, dass unter den Bedingungen der Kombination eines nukleinsäurebindenden Trägermaterials mit einer chaotropen Verbindung und einem Alkohol mit einer Konzentration von ca. 25 % dazu führt, dass bevorzugt lang- und längerkettige Nukleinsäuren binden, die kurzkettigen Nukleinsäuren dagegen nur sehr schlecht binden und sich demnach im Durchbruch/ Überstand befinden, aus welchem sie dann gemäß dem beschriebenen Verfahren isoliert werden können. Auch dieser zweite Verfahrensweg beschreibt lediglich eine Trennung von längerkettigen Nukleinsäuren. Es gibt keinen Hinweis darauf, dass man eine fraktionierte Isolierung von kurzkettigen und länger-/ langkettigen Nukleinsäuren durchführen kann. Auch erlaubt das Verfahren keine Isolierung von Gesamtnukleinsäure und eine nachfolgende Trennung von kurzkettigen und länger-/ langkettigen Nukleinsäuren. Entscheidend für die Anreicherung von kurzkettigen Nukleinsäuren ist die Kombination einer chaotropen Verbindung mit vorzugsweise Isopropanol.

In der Patentschrift DE102006045391 B4 wird ebenfalls ein Verfahren beschrieben, welches sich für die Trennung von lang- und kurzkettigen Nukleinsäuren aus einem diese Nukleinsäuren enthaltenen Gemisch eignet. Die Trennung erfolgt dabei nicht durch eine Änderung der Bindungsbedingungen, sondern über ein sukzessives Passagieren der mit einer chaotropen Verbindung in Kontakt gebrachten Probe über zwei Siliziumdioxidphasen, welche zwei unterschiedliche Porengrößen aufweisen. Nach zweimaligem Durchlaufen der Probe durch die Siliziumdioxydphase 1 und nach zweimaligem Durchlaufen der Probe über die Siliziumdioxydphase 2 sind die Nukleinsäurefragmente in der Form getrennt, als das an der ersten Phase Nukleinsäurefragmente, die mindestens 20.000 Basenpaare groß sind, binden und an der zweiten Phase Nukleinsäurefragmente von höchstens 10.000 Basenpaaren binden. Dieses Verfahren eignet sich damit nicht für die Trennung von kurz- und langkettigen Nukleinsäuren, wie es gerade die Zielstellung der vorliegenden Erfindung ist.

Ebenfalls zum Stand der Technik gehört die Druckschrift WO 2004/042058 A2. Dort wird offenbart, dass der pH-Wert der verwendeten Bindungspuffer einen wesentlichen Einfluss auf sowohl die Ausbeute an den zu gewinnenden Nukleinsäuren ist als auch eine Selektivität gegenüber den Fragmentlängen von z.B. aufzureinigenden PCR-Produkten besitzt. Dabei ist es nicht notwendig, mono- und multivalente Salze in einer Lösung mit einander zu kombinieren. Bevorzugt werden divalente und besonders bevorzugt Mg- oder Ca-salze verwendet. Bei Bindungspuffern ohne Alkohol, allerdings mit divalenten Kationen - erfolgt eine Rückgewinnung von DNA-Fragmenten in quantitativen Mengen und über das Größenspektrum 100 bp bis 10.000 bp bevorzugt bei einem pH-Wert von >8,5.

Der vorliegenden Erfindung lag die Aufgabe zu Grunde, die Nachteile der im Stand der Technik beschriebenen Lösungen zu beseitigen.

Die Aufgabe wurde gemäß den Merkmalen der Patentansprüche gelöst.

Erfindungsgemäß wurde ein Verfahren bereitgestellt, dass es erlaubt, aus einer Probe, welche ein Gemisch aus kurz- und längerkettigen/ langkettigen Nukleinsäuren (insbesondere DNA) enthält, selektiv unterschiedliche Größenfraktionen der DNA aufzureinigen bzw. zu isolieren und damit diese Fraktionen für weitere Anwendungen verfügbar zu haben.

Außerdem wurde ein Verfahren bereitgestellt, dass es erlaubt, aus einer Probe, welche ein Gemisch aus kurz- und länger-/langkettigen Nukleinsäuren (DNA) enthält, selektiv kurzkettige Nukleinsäuren von langkettigen Nukleinsäuren zu trennen, wobei das Größenspektrum der jeweiligen Fraktion einstellbar ist (z.B. Trennung von DNA die kleiner 500 Basenpaaren ist von DNA die größer 500 Basenpaare). Die jeweils nicht gewünschte Fraktion kann dann verworfen werden, wogegen mit der gewünschten Fraktion weitergearbeitet wird.

Weiterhin wurde ein Verfahren bereitgestellt, dass es erlaubt, aus einer großvolumigen Probe (z.B. Plasma, Serum, Urin oder anderen zellfreien Körperflüssigkeiten) kurzkettige und länger-/langkettige Nukleinsäuren aufzukonzentrieren und nachfolgend wie unter Punkt 1 bzw. Punkt 2 beschrieben, eine selektive größenfraktionierte Trennung von Nukleinsäuren durchzuführen.

Der vorliegenden Erfindung lag eine Beobachtung zu Grunde, dass die Isolierung von kurzkettigen DNA- Fragmenten (DNA-Fragmente kleiner als 500 bp) aus einem PCR-Reaktionsansatz immer problematischer ist, wenn der verwendete Amplifikationspuffer einen hohen pH-Wert aufweist und wenn für die Aufreinigung eine Lösung eines chaotropen Puffers eingesetzt wird.

Die Ursache scheint darin zu liegen, dass ein höherer pH-Wert deutlich die Bindung insbesondere von kurzkettigen Nukleinsäuren an mineralische Trägermaterialien negativ beeinflusst. Sollte dies die Ursache sein, dann würde die Möglichkeit bestehen, über die Änderung des pH-Wertes Bindungsbedingungen einstellen zu können, die eine selektive Bindung von kurzkettigen bzw. längerkettigen/ langkettigen Nukleinsäuren erlauben könnte.

Im Unterschied zum Verfahren, welches in der Druckschrift WO 2004/042058 A2 beschrieben wurde, sind weder di- oder multivalene Kationen noch eine Kombination von mono- und divalenten Kationen für die Isolierung der unterschiedlich langen Nukleinsäuren notwendig.

Mit dem erfindungsgemäßen Verfahren kann genau dies sehr effizient erreicht werden. Darüber hinaus können die Bindungsbedingungen so flexibel eingestellt werden, dass de facto jede beliebige Größenfraktionierung von Nukleinsäuren vorgenommen werden kann. Das erfindungsgemäße Verfahren erlaubt damit, aus einer Probe, welche ein Gemisch aus kurz- und längerkettigen/ langkettigen Nukleinsäuren (DNA) enthält, selektiv unterschiedliche Größenfraktionen der DNA aufzureinigen bzw. zu isolieren oder auch bestimmte Größenfraktionen selektiv zu entfernen. Der Ablauf des Verfahrens basiert darauf, dass ein Gemisch aus kurz- und längerkettigen/ langkettigen Nukleinsäuren mit einer Lösung, welche ein chaotropes Salz enthält, in Kontakt gebracht wird. Um eine selektive Anbindung von kurzkettigen oder längerkettigen/ langkettigen Nukleinsäuren zu erreichen, wird der pH-Wert dieses Gemisches variabel eingestellt. Es zeigt sich, dass bei einem pH-Wert von 6 oder 7 sowohl kurzkettige als auch längerkettige/ langkettige Nukleinsäuren sehr effizient an ein mineralisches Trägermaterial (z.B. Glasfaserfiltermaterial) binden. Nach der Bindung der Nukleinsäuren an ein mineralisches Material (z.B. Zentrifugationssäule mit Glasfasermaterial) wird das Trägermaterial mit Waschpuffern gewaschen und final nach Zugabe von Wasser oder eines anderen Niedrigsalzpuffers die gebundene Nukleinsäurefraktion vom Träger eluiert. Überraschenderweise zeigt sich, dass eine Erhöhung des pH-Wertes des Gemisches der Probe und der wässrigen Lösung eines chaotropen Salzes das Größenspektrum der Nukleinsäuren, welche am Träger binden, verändert. Eine sukzessive Erhöhung des pH-Wertes bewirkt, dass zuerst kurzkettige Nukleinsäuren nicht mehr binden und nachfolgend auch längerkettige/ langkettige Nukleinsäuren nicht binden und lediglich langkettige Nukleinsäuren am Träger verbleiben. Diese Fraktionierung kann dabei flexibel eingestellt werden. Letztlich binden dann am Trägermaterial längerkettige/ langkettige oder auch nur langkettige Nukleinsäuren und kurzkettige bzw. kurzkettige/ längerkettige Nukleinsäuren nicht. Die gebundenen Nukleinsäuren können dann aufgereinigt und isoliert werden. Von weitaus größerem Interesse sind aber gerade die Anwendungen, die darauf abzielen, längerkettige/ langkettige Nukleinsäuren aus einem Gemisch, welches kurzkettige sowie längerkettige/ langkettige Nukleinsäuren enthält, abzureichern und die kurzkettigen Nukleinsäuren zu isolieren. Gerade diese Zielstellung kann mit dem erfindungsgemäßen Verfahren einfach und effizient erreicht werden. Nach der Einstellung des pH-Wertes im Gemisch aus Probe und chaotroper Lösung binden längerkettige/ langkettige Nukleinsäuren an ein erstes Trägermaterial. Die nichtgebundene Fraktion an Nukleinsäuren befindet sich dann jeweils im Filtrat.

Die Isolierung der im Filtrat befindlichen kurzkettigen Nukleinsäuren erfolgt mittels des erfindungsgemäßen Verfahrens wie folgt. Das Filtrat wird jetzt mit einer wässrigen Lösung versetzt, deren pH-Wert niedriger ist, als der pH-Wert, welcher in der Lösung vorliegt, die für die erste Bindungsreaktion eingesetzt wurde. Dies kann durch die Zugabe eines nichtionischen Tensids, z.B. aus der Klasse der Alkylglycoside oder Oktylphenolethoxylate, oder mit einen Alkohol oder ein Gemisch aus nichtionischem Tensid und Alkohol oder mit einem Gemisch aus einer chaotropen Verbindung und einem nichtionischen Tensid etc erfolgen. Wichtig ist dabei nur, dass die resultierenden Bindungsbedingungen so geändert werden, dass die im Filtrat enthaltene Nukleinsäurefraktion effizient an ein zweites Trägermaterial bindet.

Danach wird das Gemisch mit einem zweiten nukleinsäurebindenden Träger in Kontakt gebracht (z.B. eine Zentrifugationssäule mit Glasfasermaterial), dann gewaschen und final die Nukleinsäure durch Zugabe von Wasser oder eines Niedrigsalzpuffers vom Träger eluiert.

Es zeigt sich, dass die Bindungsbedingungen so geändert werden, dass jetzt die im Filtrat enthaltenen kurzkettigen Nukleinsäuren oder ggf. die kurzkettigen/ längerkettigen Nukleinsäuren effizient an das Trägermaterial binden - die Nukleinsäurefraktion, welche nicht am ersten Trägermaterial gebunden wurde. Damit basiert die vorliegende Erfindung auf einem variablen Wechselspiel der flexiblen Änderung der Bindungsbedingungen, welche für die Adsorption von Nukleinsäuren an einem ersten Trägermaterial und den Bindungsbedingungen, welche für die Adsorption von Nukleinsäuren am zweiten Trägermaterial vorliegen. Die Bindungsbedingungen für das erste Trägermaterial werden so gestaltet, dass je nach Wunsch kurzkettige oder längerkettige oder langkettige Nukleinsäuren an das erste Trägermaterial binden. Die nichtgebunden Fraktionen der Nukleinsäuren befinden sich dann nach Durchführung des Extraktionsschrittes im Filtrat. Die Bindungsbedingungen werden jetzt durch Zugabe von Lösungenso eingestellt, dass diese Nukleinsäuren jetzt an das zweite Trägermaterial binden.

Überraschenderweise zeigt sich auch, dass es sich bei der Größenfraktionierung nicht um eine prozentuale Anreicherung/ Abreicherung bestimmter Fragmentgrößen um 20%, 50% oder 60 % handelt, wie dies in der zitierten Druckschrift EP2128169A1 beschrieben ist, sondern das diese Trennung auch fast quantitativ (zu 99%) erreicht werden kann.

Mittels des erfindungsgemäßen Verfahrens können somit selektiv und flexibel zwei Nukleinsäurefraktionen getrennt isoliert werden und liegen parallel vor. Möchte man nicht beide Fraktionen haben, sondern nur eine Fraktionen, dann kann das jeweilige Trägermaterial verworfen werden und man arbeitet nur mit dem Trägermaterial, an welchem sich die gewünschte Nukleinsäurefraktion befindet. Somit kann man die gewünschte Anreicherung/ Abreicherung einer ausgewählten Nukleinsäuregrößenfraktion einfach und effizient einstellen und realisieren.

Die vorliegende Erfindung der selektiven Größenfraktionierung von Nukleinsäuregemischen erlaubt es aber auch, eine biologische Probe, welche unterschiedliche Nukleinsäurefraktionen enthält, direkt zu bearbeiten. Insbesondere zellfreie Körperflüssigkeiten (Serum, Plasma, Urin, etc.) sind wichtige Ausgangsproben, da man aus ihnen effizient sog. zirkulierende zellfreie DNA isolieren möchte. Wie schon aufgeführt, ist es hier wichtig z.B. langkettige Nukleinsäuren aus der Probe abzureichern und kurzkettige Nukleinsäuren effizient aufzureinigen (z.B. für eine Prenataldiagnostik eine Trennung von maternaler und fötaler DNA zu erreichen). Das erfindungsgemäße Verfahren ermöglicht auch die direkte Bearbeitung solcher Proben. Hierzu wird die Probe mit einem Lyse-/ Bindungspuffer versetzt, welcher aus der erfindungsgemäßen Kombination einer chaotropen Lösung mit einem spezifisch und flexibel eingestellten pH-Wert besteht. Auch hierbei können durch die Veränderungen des pH-Wertes (je höher der pH-Wert bei einer konstanten Salzkonzentration, desto weniger effizient binden kurzkettige bzw. danach folgend längerkettige und langkettige Nukleinsäuren) so flexibel eingestellt werden, dass eine gewünschte Größenfraktionierung definiert werden kann. Nach kurzer Lyse der Ausgangsprobe wird die Probe mit einem nukleinsäurebindenden Träger (z.B. Zentrifugationssäule mit Glasfasermaterial) in Kontakt gebracht. Dabei binden die längerkettigen/ langkettigen Nukleinsäuren an diesen Träger, die kurzkettigen befinden sich im Filtrat. Die Zentrifugationsäule kann jetzt verworfen werden oder wird gemäß dem schon beschriebenen Verfahrensablauf gewaschen und die gebundenen längerkettigen/ langkettigen Nukleinsäuren werden vom Träger eluiert und liegen dann für weitere Anwendungen vor. Das Filtrat wird dann mit einer weiteren Lösung so behandelt, dass der resultierende pH-Wert jetzt niedriger als der pH-Wert des Filtrates vor der Zugabe dieser Lösung war. Dies kann, wie schon aufgeführt, durch die Zugabe eines Tris-Puffers oder durch die Zugabe einer Salzlösung oder eines Detergenzes oder eines Alkohols oder auch aus Mischungen dieser Komponenten erfolgen. Diese Lösung ermöglicht nachfolgend die Anbindung der im Filtrat enthaltenen Nukleinsäuren an das zweite Trägermaterial.

Nach Waschschritten werden die kurzkettigen Nukleinsäuren eluiert und können analysiert werden. Mittels des erfindungsgemäßen Verfahrens ist es somit möglich, die längerkettigen/ langkettigen Nukleinsäuren aus der Probe effizient abzureichern und die kurzkettigen Nukleinsäuren effizient zu isolieren. Besonders bedeutsam erweist sich das erfindungsgemäße Verfahren dadurch, dass es auch möglich ist, großvolumige biologische Proben so zu bearbeiten, dass eine Größenfraktionierung von Nukleinsäuren möglich ist. Hierbei ist wiederum die Untersuchung von zellfreien Körperflüssigkeiten von steigendem Interesse. Wie bereits beschrieben, sind zirkulierende Nukleinsäuren sehr interessant für eine Vielzahl von diagnostischen Fragestellungen. Allerdings ist die Menge dieser zellfreien zirkulierenden DNA sehr gering, so dass die Möglichkeit der Bearbeitung großer Probenvolumen sehr wünschenswert ist, da damit eine deutliche Erhöhung der Ausbeuten an DNA erreicht werden kann. Die Offenlegungsschrift (WO2009/055596) beschreibt ein effizientes und sehr einfaches Verfahren zur Anreicherung von zellfreier DNA aus großvolumigen Proben. Für diese Anreicherung und nachfolgende Aufreinigung zellfreier DNA gibt es auch einen kommerziell verfügbaren Kit (PME free-circulating DNA Extraction Kit; Analytik Jena AG). Allerdings gestattet dieses Verfahren nur die Aufreinigung der zellfreien zirkulierenden Gesamt-DNA. Es erlaubt keine Trennung von kurzkettigen und langkettigen Nukleinsäuren. Hier bietet die vorliegende Erfindung eine Lösung an. So ist es möglich, den Anreicherungsschritt für zellfreie DNA mit einer nachfolgenden Größenfraktionierung von kurzkettigen und längerkettigen/ langkettigen Nukleinsäuren zu kombinieren. Damit besteht erstmalig die Möglichkeit, große Probenvolumina zu bearbeiten und auch eine Größenfraktionierung zu ermöglichen. Für die nachfolgende Durchführung des erfindungsgemäßen Verfahrens erfolgt dabei in einem ersten Schritt die Anreicherung der zellfreien Gesamt-DNA einer Probe. Dazu gibt man zur Probe eine wässrige Alginatlösung und eine wässrige Lösung, enthaltend Salze von di- bzw. polyvalenten Kationen (z.B. Kalziumchlorid oder Aluminiumchlorid). Nach kurzer Zentrifugation wird der großvolumige Überstand entfernt und mit dem resultierenden Pellet wird weitergearbeitet. In diesem Pellet befindet sich die zellfreie Gesamt-DNA.

Das Pellet wird jetzt mit einem Puffer aufgelöst und nachfolgend die Bedingungen eingestellt, die eine effiziente Adsorption der angereicherten zellfreien Gesamt-DNA an ein erstes Trägermaterial erlauben (z.B. Zentrifugationssäule mit Glasfasermaterial). Nach Waschschritten wird über dieses erste Trägermaterial die zellfreie Gesamt-DNA eluiert. Im resultierenden Eluat befinden sich damit kurzkettige und längerkettige/ langkettige Nukleinsäuren. Von diesem Eluat wird ein kleines Volumen aufgehoben. Das restliche Volumen wird jetzt gemäß dem bereits beschriebenen erfindungsgemäßen Verfahren weiter bearbeitet. Zum Eluat wird eine chaotrope Lösung gegeben, deren pH-Wert entsprechend der Erfindung so eingestellt wird, dass eine gewünschte Nukleinsäurefraktion (längerkettige/ langkettige Nukleinsäure) an das zweite Trägermaterial bindet (durch die Einstellung des pH-Wertes wird festgelegt, welche Fragmentlängen der Gesamt-DNA an das Trägermaterial binden bzw. welche Fraktion nicht bindet und deshalb sich dann im Filtrat befindet). Nachfolgend wird dieser Ansatz mit einem nukleinsäurebindenden Träger (z.B. eine Zentrifugationssäule mit einem Glasfasermaterial) in Kontakt gebracht. Danach wird das Trägermaterial mit Waschpuffern gewaschen und final nach Zugabe von Wasser oder eines anderen Niedrigsalzpuffers die gebundene Nukleinsäurefraktion vom Träger eluiert. Dieses zweite Eluat enthält erfindungsgemäß jetzt die Fraktion der längerkettigen/ langkettigen Nukleinsäure. Die Isolierung der im Filtrat befindlichen kurzkettigen Nukleinsäuren erfolgt dann wiederum durch Zugabe einer Lösung, welche einen niedrigeren pH-Wert aufweist, als der pH-Wert, welcher für die vorherige Anbindung an das Trägermaterial vorlag. Jetzt wird gemäß der Erfindung sichergestellt, dass die im Filtrat befindlichen Nukleinsäuren effizient an ein drittes Trägermaterial binden und isoliert werden können.

Nachfolgend wird das Gemisch mit einem dritten nukleinsäurebindenden Träger in Kontakt gebracht (z.B. eine Zentrifugationssäule mit Glasfasermaterial), dann gewaschen und final die Nukleinsäure durch Zugabe von Wasser oder eines Niedrigsalzpuffers vom Träger eluiert. Damit hat man dann in diesem Eluat die dritte Fraktion - die kurzkettigen Nukleinsäuren. Das erfindungsgemäße Verfahren erlaubt erstmals, dass aus einer Probe drei unterschiedliche Nukleinsäurefraktionen simultan und parallel bereitgestellt werden. Diese unterschiedlichen Fraktionen können jetzt untersucht werden

Die Erfindung wird nachfolgend anhand von Ausführungsbeispielen erklärt.

Diese Ausführungsbeispiele stellen dabei aber keine Limitierung der Erfindung dar.

### Erläuterung der Figuren

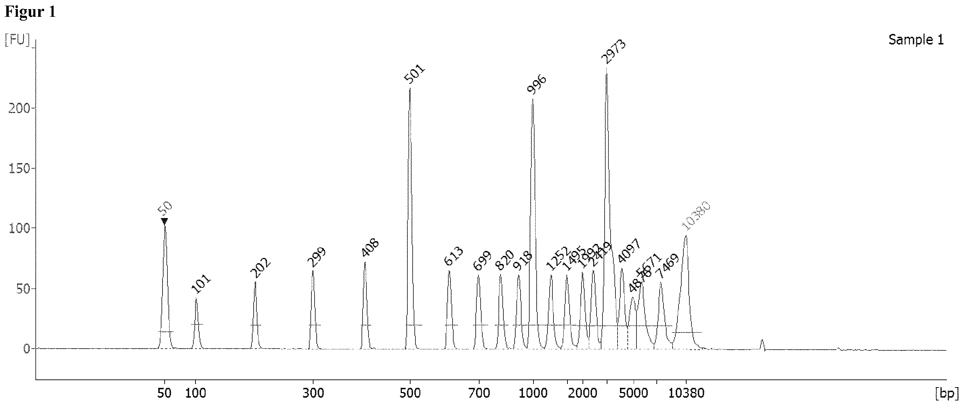

Figur 1 zeigt eine Agilent Bioanalyzer Analyse (Probe + chaotrope Salzlösung).

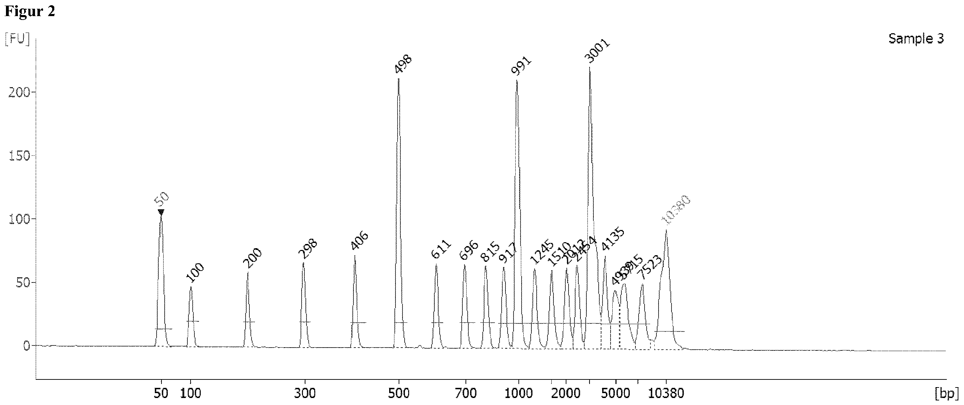

Figur 2 zeigt eine Agilent Bioanalyzer Analyse (Probe + chaotrope Salzlösung + Tris-Lösung pH 6.0).

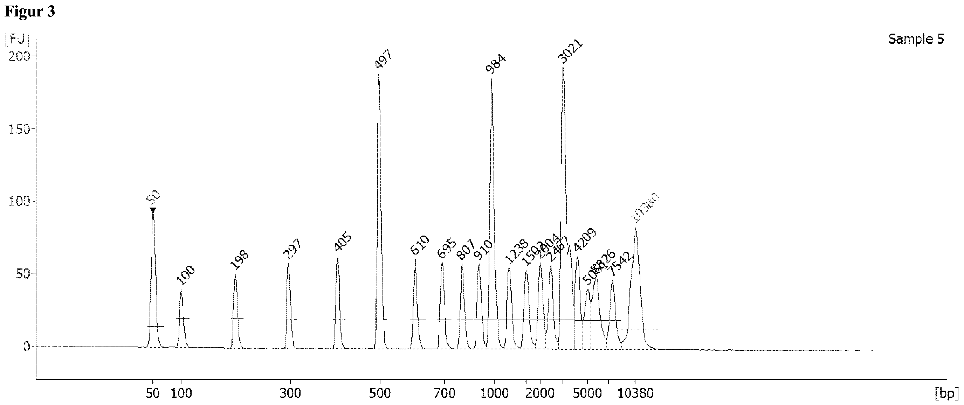

Figur 3 zeigt eine Agilent Bioanalyzer Analyse (Probe + chaotrope Salzlösung + Tris-Lösung pH 7.0).

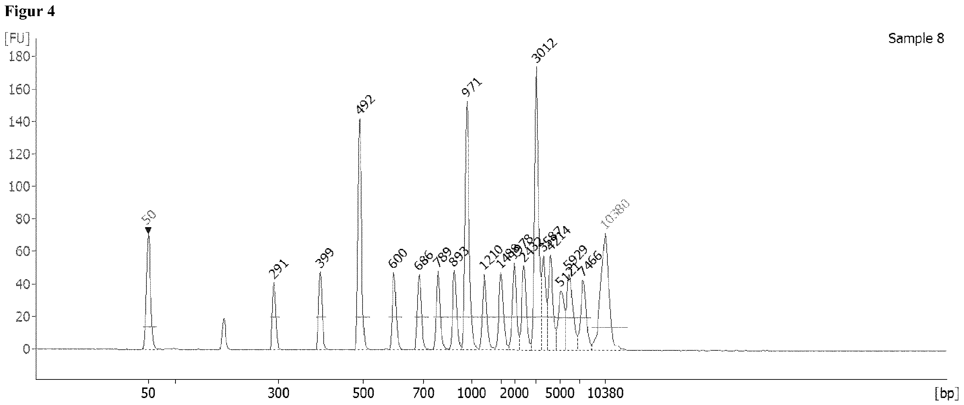

Figur 4 zeigt eine Agilent Bioanalyzer Analyse (Probe + chaotrope Salzlösung + Tris-Lösung pH 7.5).

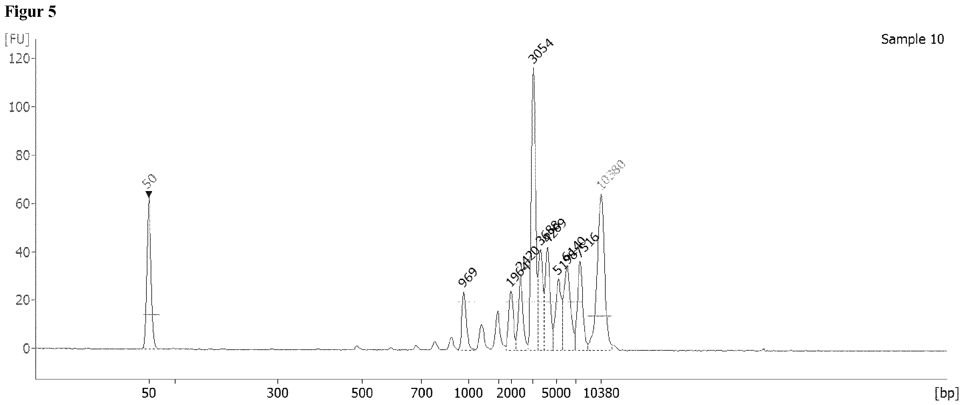

Figur 5 zeigt eine Agilent Bioanalyzer Analyse (Probe + chaotrope Salzlösung + Tris-Lösung pH 8.0).

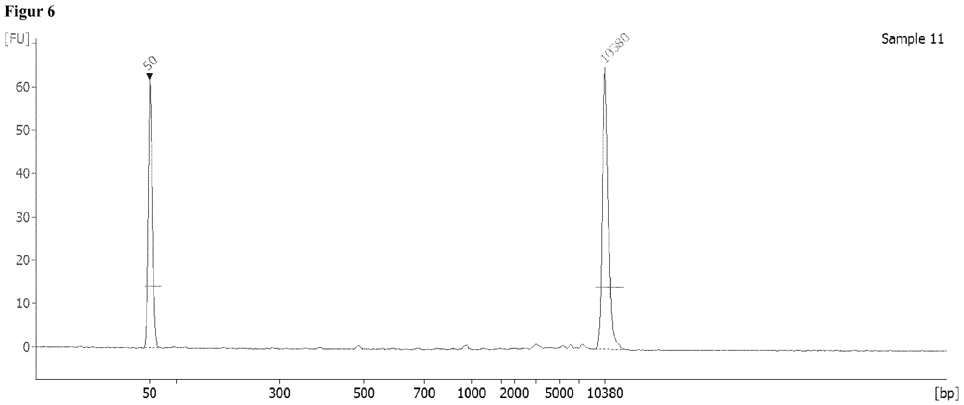

Figur 6 zeigt eine Agilent Bioanalyzer Analyse (Probe + chaotrope Salzlösung + Tris-Lösung pH 9.0).

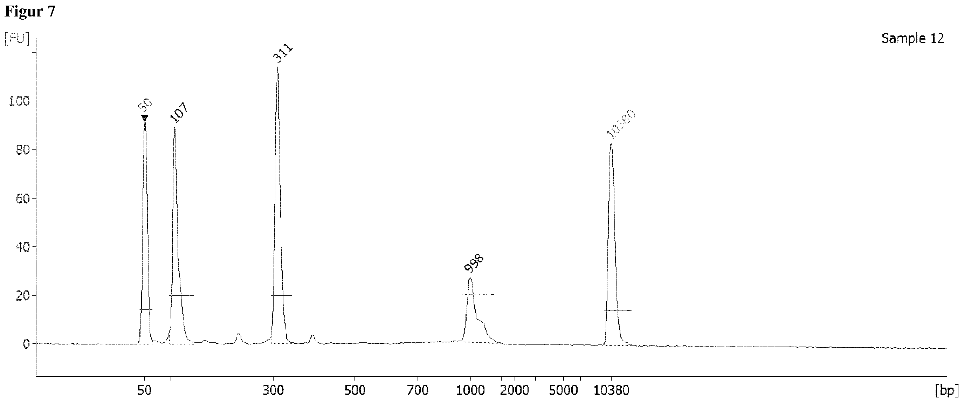

Figur 7 zeigt eine Agilent Bioanalyzer Analyse (Probe mit den drei unterschiedlichen DNA-Fragmenten).

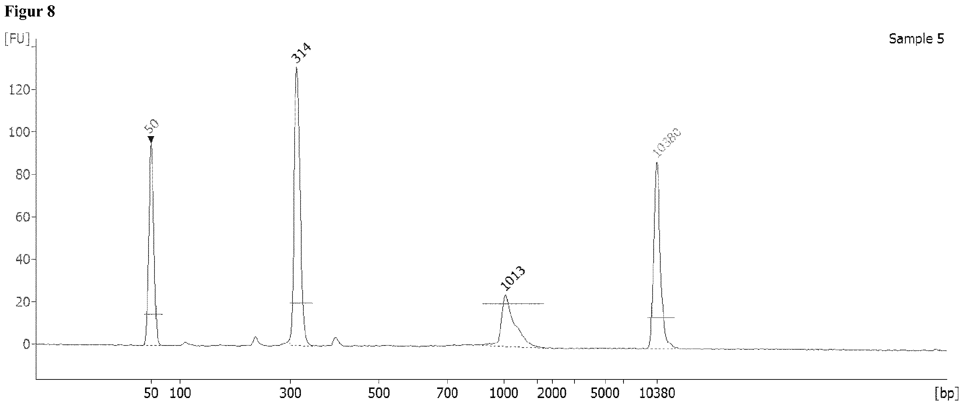

Figur 8 zeigt eine Agilent Bioanalyzer Analyse (Eluat der ersten Fraktion mit den längerkettigen DNA-Fragmenten).

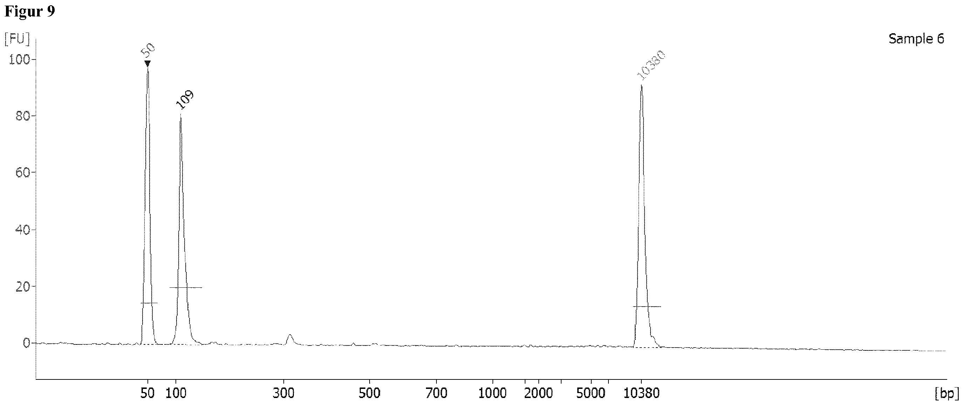

Figur 9 zeigt eine Agilent Bioanalyzer Analyse (Eluat der zweiten Fraktion mit dem kurzkettigem DNA-Fragment).

### Ausführungsbeispiel 1

Selektive größenfraktionierte Isolierung von DNA direkt aus einer Plasmaprobe

Ziel des Beispiels war es, zu zeigen, dass auch aus einer biologischen Probe direkt eine größenfraktionierte Isolierung von DNA möglich ist, d.h. das es effizient möglich ist , kurzkettige Nukleinsäuren von längerkettigen/ langkettigen Nukleinsäuren zu trennen und beide Fraktionen parallel aufzuarbeiten bzw., wenn nur eine Fraktion gewünscht ist, die andere Fraktion aus der Probe effizient zu entfernen, damit diese keinen störenden Einfluss auf spezifische Applikationen hat.

Ausgangsprobe waren jeweils 200 µl einer Plasmaprobe, enthaltend kurzkettige Nukleinsäurefragmente (51 bp, 77 bp, 103 bp, 149, bp, 199, bp, 298, bp) sowie ein längerkettiges Fragment von 1118 bp. Es sollte gezeigt werden, dass die kurzkettige Fraktion von der längerkettigen Fraktion getrennt werden kann.

Die Proben wurden mit 300 µl einer chaotropen Verbindung (4M Guanidinisothyocyanat, 5 mM Tris HCl, pH 7.5) versetzt. Der Probe wurde weiterhin ein nichtionischen Tensid aus der Klasse der Alkylglucoside und 20 µl Proteinase K (20 mg/ ml) zugegeben. Die Probe wurde für 15 min bei 70°C lysiert. Zur Einstellung des für die vorgesehene selektive Trennung der Nukleinsäurefragmente notwendigen pH-Wertes wurde der Lösung 4 µl einer Tris-HCl-Lösung pH 8 zugegeben. Danach wurde der Ansatz über eine erste Zentrifugationssäule (mit Glasfasermaterial) zentrifugiert. Das Filtrat wurde mit 400 µl eines Gemisches aus einer chaotropen Verbindung und eines nichtionischen Tensids aus der Klasse der Alkylglucoside (4M Guanidinisothyocyanat/ 30 % nichtionisches Tensid) versetzt, um die spezifischen Bindungsbedingungen für die Adsorption der im Filtrat befindlichen kurzkettigen Nukleinsäurefragmente herzustellen. Dieser Ansatz wurde auf eine zweite Zentrifugationssäule (mit Glasfasermaterial) überführt und zentrifugiert.

Beide Zentrifugationssäulen wurden dann mit ethanolhaltigen Waschpuffern gewaschen und final die gebundene Nukleinsäure mit Wasser eluiert.

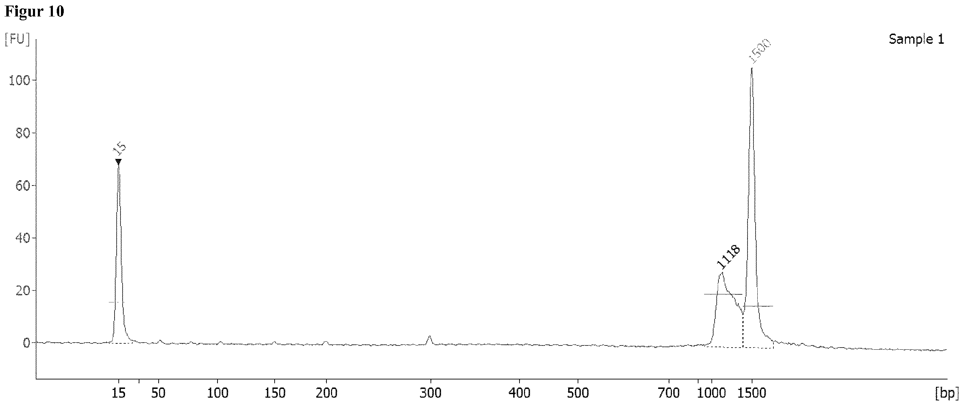

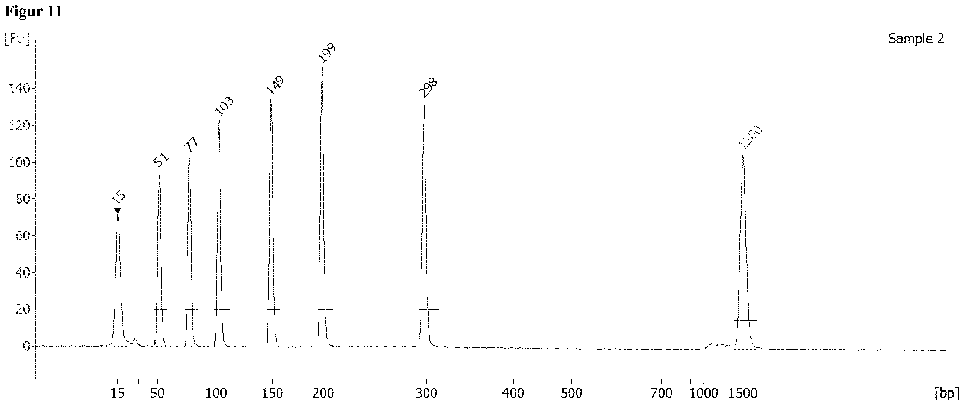

Der Nachweis der isolierten DNA-Fragmente erfolgte wiederum mittels eines Agilent Bioanalyzers unter Nutzung des DNA Kits 7500. Die Auswertung zeigt, dass eine größenfraktionierte Trennung von DNA direkt aus einer biologischen Probe möglich ist. In diesem Beispiel wurden die Bindungsbedingungen für die erste Zentrifugationssäule und für die zweite Zentrifugationssäule so eingestellt, dass an der ersten Säule Fragmente, die kurzkettigen Nukleinsäurefragmente, nicht gebunden wurden und diese kurzkettigeren Fragmente dann im Filtrat sind und über die zweite Zentrifugationssäule isoliert werden können. Somit konnte gezeigt werden, dass man längerkettige Nukleinsäuren effizient aus einer Probe entfernen kann bzw. je nach Zielstellung beide Fraktionen parallel aufgearbeitet werden und verfügbar sind. Die Agilent Bioanalyzer Analysen sind auf den nachfolgenden Figuren 10 und 11 dargestellt.

Figur 10 zeigt eine Agilent Bioanalyzer Analyse (Eluat der ersten Fraktion mit dem längerkettigen DNA-Fragment).

Figur 11 zeigt eine Agilent Bioanalyzer Analyse (Eluat der zweiten Fraktion mit den kurzkettigem DNA-Fragmenten).

### Ausführungsbeispiel 2

Anreicherung von zellfreier DNA aus Human-Plasmaprobe und nachfolgende selektive Trennung der kurzkettigen und längerkettigen/ langkettigen Nukleinsäuren, differenzierte Isolierung der beiden Fraktionen und Messung der Abreicherung der kurzkettigen Nukleinsäuren von den längerkettigen/ langkettigen Nukleinsäuren mittels realTime PCR. Darstellung des Einflusses einer sukzessiven Erhöhung des pH-Wertes für die selektive Bindung an die erste Filtersäule und das korrespondierende Filtrat.

Ausgangsmaterial war eine 1 ml Human-Plasmaprobe. Die Anreicherung der DNA-Fragmente erfolgte mittels eines kommerziell verfügbaren Kits (PME free-circulating DNA Extraction Kit; Analytik Jena AG) nach Herstellerangaben. Nach Anreicherungsschritt wurde die zellfreie Gesamtnukleinsäure gemäß Herstellerangaben isoliert.

Das Eluat mit der isolierten Gesamtnukleinsäure wurde dann für die selektive Trennung der kurzkettigen von der längerkettigen/ langkettigen Nukleinsäurefraktion eingesetzt. Es wurden 4 separate Ansätze getestet. Die Bindungsbedingungen wurden so gewählt, dass an die erste Zentrifugationsäule durch eine sukzessive Erhöhung des pH-Wertes immer weniger effizient auch die längerkettigen Nukleinsäuren binden sollten, so dass sich dadurch dann auch längerkettige Nukleinsäuren im Filtrat befinden und letztlich diese dann nachfolgend an die zweite Säule binden sollten.

Das nach Anreicherung und Extraktion erhaltene Eluat (40 µl) mit der Gesamtnukleinsäure an zellfreien DNA wurde mit 400 µl einer chaotropen Verbindung (4M Guanidinisothyocyanat; 5 mM Tris HCl, pH 7.5) gemischt. Zur Beeinflussung des pH-Wertes erfolgte die Zugabe von 1 µl 0.1N NaOH (Probe 1), 5 µl 0.1N NaOH (Probe 2), 10 µl 0.1N NaOH (Probe 3), 20 µl 0.1N NaOH (Probe 4).

Danach wurde der Ansatz über eine erste Zentrifugationssäule (mit Glasfasermaterial) zentrifugiert. Die Zentrifugationssäule wurde aufbewahrt. Zur Isolierung der kurzkettigen DNA wurde das Filtrat mit 400 µl eines Gemisches aus einer chaotropen Verbindung und eines nichtionischen Tensids aus der Klasse der Alkylglucoside (4M Guanidinisothyocyanat/ 30 % nichtionisches Tensid, 50 mM Tris HCl, 6.0) versetzt und dieser Ansatz auf eine zweite Zentrifugationssäule (mit Glasfasermaterial) überführt und zentrifugiert. Nachfolgend wurden die beiden Zentrifugationssäulen mit einem alkoholischen Waschpuffer gewaschen, durch einen Zentrifugationsschritt getrocknet und die gebundene Nukleinsäure durch Zugabe von Wasser eluiert.

Die eluierten Nukleinsäurefraktionen wurde dann in einer realTime PCR getestet.

Dazu wurde eine SybrGreen-basierte realTime PCR zur Amplifizierung eines ca. 1 kb großen Cytochrom b Fragmenten durchgeführt. Jeweils beide Eluatfraktionen aus den beiden Proben wurden für die PCR eingesetzt.

Verwendete PCR Primer:
CyB S: CCA GCY CCA TCA AAC ATC TCA KCA TGA TG
CyB AS: TTG GCT GAG TGG TCG GAA TAT TAT GCT KCG TTG YTT
Reaktionsansatz (Amplifikation/ Hybridisierung)
Pro Probe:

| | |
|---|---|
| sense Primer (50 pmol/ µl) | 0,1 µl |
| antisense Primer (50 pmol/ µl) | 0,1 µl |
| SyGreen MasterMix (AJ) | 7,5 µl |
| PCR-Grade H₂O | add. 15 µl |

Die PCR wurde in einem handelsüblichen realTime PCR-Cycler durchgeführt: Amplifikationskonditionen

| | | |
|---|---|---|
| Schritt 1: | Denaturierung | 95°C 120" |
| Schritt 2 | Amplifizierung | 45 Zyklen |
| | | 95°C 4" |
| | | 55°C 40" |

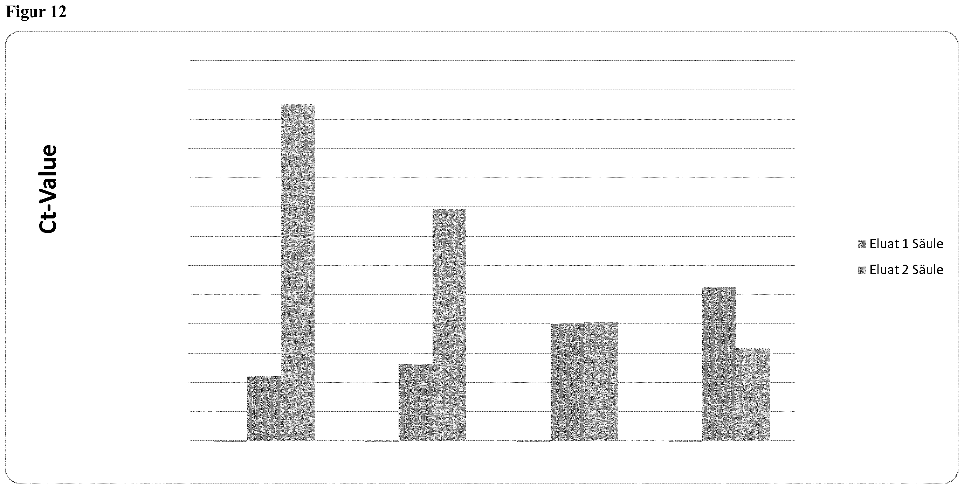

Anschließend wurde eine Schmelzkurvenanalyse durchgeführt, um die Amplifikationsspezifität nachzuweisen. Die PCR-Ergebnisse sind in der Fig.12 grafisch dargestellt. Je kleiner die Ct-Werte, desto höher ist der Anteil der jeweiligen Nukleinsäurefraktion in der Probe. Es ist deutlich zu sehen, wie effizient längerkettige Nukleinsäuren aus der kurzkettigen Fraktion entfernt werden können.

So ist der Anteil von längerkettigen Nukleinsäuren in der Probe 1 gerade mal noch bei 1%, d.h. man hat eine Abreicherung von mehr ca. 99%. Es ist auch deutlich zu sehen, wie sich die Anteile der längerkettigen Nukleinsäuren in die Fraktion 2 verschieben, wenn man sukzessive den pH-Wert für die Bindung an die erste Säule erhöht.

Figur 12 zeigt die Darstellung der Ct-Werte.

### Definitionen:

### Chaotrope Substanten bzw. chaotrope Salze:

Substanzen, die regelmäßige - auf der Bildung von Wasserstoffbrückenbindungen beruhende - Struktur von flüssigem Wasser zerstören, indem sie die Bildung der zur Solvatation notwendigen H2O-Käfigstrukturen verhindern. Beispiele für chaotrope Bestandteile sind Thiocyanate, Iodide oder Perchlorate. Sie bewirken die Denaturierung von Proteinen, die Erhöhung der Löslichkeit unpolarer Substanzen in Wasser sowie Zerstörung der hydrophober Wechselwirkung.

### Aliphatische Alkohole

Aliphatische Alkohole im Sinne dieser Patentbeschreibung und Patentansprüche sind alle Alkohole, die ihre OH-Gruppe an einem aliphatischen C-Atom tragen, mit Ausnahme von Amino-Alkoholen wie TRIS.

## Patentansprüche

1. Verfahren zur fraktionierten größenabhängigen Isolierung von Nukleinsäuren aus einem Gemisch von Nukleinsäuren mit einer unterschiedlichen Anzahl an Basenpaaren (bp), **gekennzeichnet durch** folgende Schritte:
a) zu einem Volumen des Gemischs von Nukleinsäuren wird - in Abwesenheit von aliphatischen Alkoholen - ein erster Bindungspuffer hinzugefügt, der mindestens ein chaotropes Salz und mindestens eine Substanz enthält, die den pH-Wert des Bindungspuffers erhöht
b) Anbinden an eine feste Phase und Abtrennen der durch Schritt a) gebundenen Nukleinsäuren
c) das Filtrat aus Schritt a) wird mit einem zweiten Bindungspuffer, der ein nichtionisches Tensid
oder mit einen Alkohol
oder ein Gemisch aus nichtionischem Tensid und Alkohol enthält,
versetzt
d) Anbinden an eine feste Phase und Abtrennen der durch Schritt c) gebundenen Nukleinsäuren
e) Waschen und Eluieren der nach Schritt a) und c) isolierten Nukleinsäuren nach bekannten Verfahren,
resultierend, dass die Nukleinsäuren, die nach Schritt a) isoliert wurden, nicht nur eine größere Anzahl von Basenpaaren aufweisen als die unter Schritt c) isolierten Nukleinsäuren, sondern dass sowohl nach Schritt a) als auch nach Schritt c) einzelne, bestimmte Nukleinsäure-Fraktionen mit einer bestimmten Anzahl von Basenpaaren isoliert werden, die im jeweils anderen Schritt nicht isoliert werden.

2. Verfahren nach Anspruch 1, **dadurch gekennzeichnet, dass** der zweite Bindungspuffer mindestens eine chaotrope Substanz enthält.

3. Verfahren nach Anspruch 1, **dadurch gekennzeichnet, dass** als chaotropes Salz 4M Guanidinisothyocyanat und als Substanz, die den pH-Wert des Bindungspuffers erhöht, NaOH verwendet wird.

4. Verfahren nach Anspruch 1, **dadurch gekennzeichnet, dass** als Alkohole aliphatische Alkohole und als nichtionisches Tensid eine Substanz aus der Klasse der Alkylglucoside oder Oktylphenolethoxylate verwendet wird.

5. Verfahren nach einem der Ansprüche 1 bis 4, **dadurch gekennzeichnet, dass** die Größe der Nukleinsäuren (Anzahl der Basenpaare), die nach Schritt 1a) isoliert werden, durch die schrittweise Erhöhung des pH-Wertes des Bindungspuffers gemäß Schritt 1a) gesteuert wird, wobei mit steigendem pH-Wert weniger kurzkettige Nukleinsäuren (geringere Anzahl an Basenpaaren) abgetrennt werden können.

6. Verfahren nach einem der Ansprüche 1 bis 5, **dadurch gekennzeichnet, dass** die Größe der Nukleinsäuren (Anzahl der Basenpaare), die aus Schritt 1c) isoliert werden, durch Zusatz eines nichtionischen Tensids oder eines Alkohols oder eines Gemischs aus nichtionischem Tensid und Alkohol zum ersten Bindungspuffer gemäß 1a) gesteuert wird, wobei mit steigender Konzentration der zugesetzten Substanzen mehr kurzkettige Nukleinsäuren (geringere Anzahl an Basenpaaren) abgetrennt werden können.

7. Verfahren nach einem der Ansprüche 1 bis 6, **dadurch gekennzeichnet, dass** vor der Fraktionierung bei einer beliebig großvolumigen Probe zunächst die Gesamt-Nukleinsäure nach einem bekannten Verfahren aufkonzentriert wird und anschließend die Nukleinsäuren gemäß einem der Ansprüche 1 bis 6 fraktioniert werden.

8. Verwendung des Verfahren nach einem der Ansprüche 1 bis 7 zur Isolierung von Nukleinsäure-Fraktionen aus einem Gemisch von Nukleinsäuren mit einer unterschiedlichen Anzahl an Basenpaaren (bp), mit folgender Größe:
a) ca. kleiner gleich 110 bp oder
b) ca. kleiner gleich 250 bp oder
c) ca. größer gleich 550 bp oder
d) größer gleich 1.000 bp

9. Verwendung des Verfahren nach einem der Ansprüche 1 bis 7 zur beliebigen Größenfraktionierung von Nukleinsäuren aus einem Gemisch aus kurz- und längerkettigen/ langkettigen Nukleinsäuren (DNA).

## Claims

1. A method for size fractioned isolation of nucleic acids from a mixture of nucleic acids with a different number of base pairs (bp), **characterized by** the following steps:
a) a first bonding buffering agent, containing at least one chaotropic salt and at least one substance, which increases the pH value of the binding buffering agent, is added to a volume of the mixture of nucleic acids, in the absence of aliphatic alcohols
b) bonding to a solid phase and separation of the nucleic acids bonded by means of step a)
c) the filtrate from step a) is mixed with a second bonding buffering agent or with a non ionic surfactant or with an alcohol or with a mixture of a non ionic surfactant and alcohol
d) bonding to a solid phase and separation of the nucleic acids bonded by means of step c)
e) washing and elution of the nucleic acids isolated after steps a) and c) according to known methods,
with the result that the nucleic acids isolated after step a) not only have a greater number of base pairs than the nucleic acids isolated in step c), but that also individual, specific nucleic acid fractions with a specified number of base pairs are isolated, both after step a) and also after step c) which fractions are not isolated in the respective other step.

2. The method according to patent claim 1, **characterized in that** the second bonding buffering agent contains at least one chaotropic substance.

3. The method according to patent claim 1, **characterized in that** 4M guanidinium isothyocyanate is used as a chaotropic salt and NaOH is used as a substance increasing the pH value of the bonding buffering agent.

4. The method according to patent claim 1, **characterized in that** aliphatic alcohols are used as alcohols and a substance from the class of alkyl glucosides or octylphenol ethoxylates are used as a non ionic surfactant.

5. The method according to patent claim 1 to 4, **characterized in that** the size of the nucleic acids (number of base pairs), which are isolated after step 1a), is controlled by gradual increase of the pH value of the bonding buffering agent according to step 1a), wherein, with increasing pH value, less short-chain nucleic acids (lower number of base pairs) can be separated.

6. The method according to any one of patent claims 1 to 5, **characterized in that** the size of the nucleic acids (number of base pairs), which are isolated from step 1c), is controlled by adding to the first bonding buffering agent according to 1a) a non ionic surfactant or an alcohol or a mixture from a non ionic surfactant and an alcohol, wherein, with increasing concentration of the substances added, more short-chain nucleic acids (lower number of base pairs) can be isolated.

7. The method according to any one of patent claims 1 to 6, **characterized in that** prior to fractionation in the case of any large volume sample at first the total nucleic acid is concentrated according to a known method, and subsequently the nucleic acids according to any one of patent claims 1 to 6 are fractionated.

8. A use of the method according to any one of patent claims 1 to 7 for the isolation of nucleic acid fractions from a mixture of nucleic acids with a different number of base pairs (bp) with the following size:
a) approx. less than or equal to 110 bp or
b) approx. less than or equal to 250 bp or
c) approx. greater than or equal to 550 bp or
d) greater than or equal to 1,000 bp.

9. The use of the method according to any one of claims 1 to 7 for any size fractionation of nucleic acids from a mixture of short chain and longer chain / long chain nucleic acids (DNA).

## Revendications

1. Procédé d'isolement fractionné selon la catégorie dimensionnelle d'acides nucléiques à partir d'un mélange d'acides nucléiques ayant un nombre différent de paires de bases (bp), **caractérisé par** les étapes suivantes:
a) on ajoute à un volume du mélange d'acides nucléiques - en l'absence d'alcools aliphatiques - un premier tampon de liaison, qui contient au moins un sel chaotrope et au moins une substance, qui augmente la valeur du pH du tampon de liaison
b) liaison à une phase solide et séparation des acides nucléiques liés par l'étape a)
c) le filtrat de l'étape a) est mélangé avec un second tampon de liaison ou avec un agent tensioactif non ionique ou avec un alcool ou avec un mélange d'un agent tensioactif non ionique et d'un alcool
d) liaison à une phase solide et séparation des acides nucléiques liés par l'étape c)
e) lavage et élution des acides nucléiques isolés après les étapes a) et c) selon des procèdes connues,
résultant en ce que les acides nucléiques, qui ont été isolés après l'étape a), présentent non seulement un plus grand nombre de paires de bases que les acides nucléiques isolés dans l'étape c), mais en ce que, aussi bien après l'étape a) qu'après l'étape c) des fractions d'acides nucléiques individuelles et déterminées sont isolées avec un nombre déterminé de paires de bases, qui ne sont pas isolées dans l'autre étape respective.

2. Procédé selon la revendication 1, **caractérisé en ce que** le second tampon de liaison contient au moins une substance chaotrope.

3. Procédé selon la revendication 1, **caractérisé en ce que** l'on utilise l'isothyocyanate de guanidine 4M comme sel chaotrope et le NaOH comme substance, qui augmente la valeur du pH du tampon de liaison.

4. Procédé selon la revendication 1, **caractérisé en ce que** l'on utilise des alcools aliphatiques comme alcools et une substance de la classe des alkylglucosides ou des éthoxylates d'octylphénol comme agent tensioactif non ionique

5. Procédé selon l'une quelconque des revendications 1 à 4, **caractérisé en ce que** la taille des acides nucléiques (nombre de paires de bases), qui sont isolés après l'étape 1a), est contrôlée par l'augmentation progressive de la valeur du pH du tampon de liaison selon l'étape 1a), dans lequel, moins d'acides nucléiques à chaîne courte (nombre de paires de bases plus faible) peuvent être séparés lorsque la valeur du pH augmente.

6. Procédé selon l'une quelconque des revendications 1 à 5, **caractérisé en ce que** la taille des acides nucléiques (nombre de paires de bases), qui sont isolés par l'étape 1c) est contrôlée par l'ajout d'un agent tensioactif non ionique ou d'un alcool ou d'un mélange d'agent tensioactif non ionique et d'alcool au premier tampon de liaison selon 1a), dans lequel une concentration croissante des substances ajoutées permet de séparer plus d'acides nucléiques à chaîne courte (nombre plus faible de paires de bases).

7. Procédé selon l'une quelconque des revendications 1 à 6, **caractérisé en ce qu'**avant le fractionnement, pour un échantillon de volume quelconque, on concentre d'abord l'acide nucléique total selon un procédé connu, puis on fractionne les acides nucléiques selon l'une quelconque des revendications 1 à 6.

8. Utilisation du procédé selon l'une quelconque des revendications 1 à 7 pour isoler des fractions d'acides nucléiques à partir d'un mélange d'acides nucléiques ayant un nombre différent de paires de bases (bp), avec la taille suivante :
a) environ inférieure ou égale à 110 bp ou
b) environ inférieure ou égale à 250 bp ou
c) environ égale ou supérieure à 550 bp ou
d) égale ou supérieure à 1000 bp.

9. Utilisation du procédé selon l'une quelconque des revendications 1 à 7 pour tout fractionnement selon la catégorie dimensionnelle d'acides nucléiques à partir d'un mélange d'acides nucléiques (ADN) à chaîne courte et à chaîne plus longue/longue.
